# EUROPEAN PATENT SPECIFICATION

(11) **EP 3 671 201 B1**
(45) Date of publication and mention of the grant of the patent: **14.12.2022**
(21) Application number: 18306777.6
(22) Date of filing: 20.12.2018
(51) Int. Cl.: G01N 29/04, G01N 27/28, F17D 5/06, G06N 20/20, G01M 3/24, G01M 3/28, G06N 5/00, G06N 3/12

(54) **AN IMPROVED METHOD FOR EVALUATING PIPE CONDITION**
VERBESSERTES VERFAHREN ZUR BEURTEILUNG EINES ROHRZUSTANDS
PROCÉDÉ AMÉLIORÉ POUR L'ÉVALUATION DE L'ÉTAT D'UNE CANALISATION

(43) Date of publication of application: 24.06.2020
(73) Proprietor: Suez International, 92040 Paris La Défense Cedex (FR)
(72) Inventor: CLAUDIO, Karim, 08036 Barcelona (ES); VAN BECELAERE, Thomas, 75012 Paris (FR); FAY, Gilles, 75019 Paris (FR)
(74) Representative: Atout PI Laplace

(56) References cited:
- EP-A1- 3 112 959
- US-A- 4 998 208
- US-B2- 7 043 373

## Description

### FIELD OF THE INVENTION

The present invention relates to the field of pipe management for a network of distribution of fluid. More specifically, it relates to the assessment of pipe condition.

### BACKGROUND PRIOR ART

Fluids, for example water, oil or gas are usually delivered through large networks that contain a large number of pipes. Over time, the pipes are subject to degradations, for example due to corrosion. Sever pipe conditions may lead to leaks in the network. It is therefore desirable to evaluate pipe condition, in order to repair or replace damaged pipes before they break or create leaks.

Pipe can be inspected directly. A number of direct inspections methods exist. For example, the pipes can be inspected visually. Pipes can also be inspected using measurements. For example, an electromagnetic flux can be applied to a pipe and analyzed. It is also possible to perform acoustical measurements that consist in sending acoustical signals and analyzing the response of various portions the pipe to the acoustical signal in order to determine their thickness.

These methods all provide the disadvantage of requiring the displacement of an operator, or local measurements. A regular inspection of a whole networks thus is, in practice, impossible and too costly to efficiently determine pipe condition of a whole large network.

A solution therefore consists in evaluating a priori the most critical pipes to inspect, in order to focus on the pipes that shall be in the most severe conditions. However, it is difficult to determine a priori which pipe is in the most severe condition, and thus should be inspected first.

The patent US 9,128,019 discloses a method that consists in evaluating in the same time a condition and a deterioration rate of a pipe. Therefore, this method allows in advance a determination of a time at which the pipe condition is expected to become critical, and at which the pipe should be inspected/repaired/replaced. However, this method also requires the inspection, at least once, of all the pipes of the network by an operator.

US 4,998,208 A discloses another method of estimating the condition of pipes based on grouping of pipes according to their corrosion environment.

There is therefore the need for a method that allows an accurate estimation of pipe condition, even of pipes that have not been inspected, while requiring only the inspection of a small portion of the pipes of the network.

### SUMMARY OF THE INVENTION

To this effect, the invention discloses a computer-implemented method comprising: a first step of clustering pipe sections of a pipe network into a number of classes, based on pipe parameters relative to the structure or to the environment of the pipe sections; and, for each class of said number of classes: a second step of extracting a sample of pipes sections of the class comprising: a first sub-step of initializing a set of candidate samples of pipe sections; a second sub-step of iteratively modifying said set of candidate samples using: a genetic algorithm based on an objective function comprising a minimization of the difference of average pipe parameters of the pipe sections of the sample, and the average pipe parameters of the pipe sections of the class; a third sub-step of selecting the candidate sample that optimizes said objective function ; a third step of obtaining, for each pipe section sample, one or more pipe condition scores determined by a condition assessment procedure; a fourth step of performing an estimation of one or more pipe condition scores for pipe sections that do not belong to the sample based on said pipe parameters, said estimation being parameterized with the pipe condition scores and pipe parameters of the pipe sections of the sample extracted at the second step.

Such pipe parameters may for example comprise one or more of: structural parameters (length of the pipe; material of the pipe; diameter of the pipe; age of the pipe...), or environmental parameters (temperatures of the environment of the pipe; humidity of the environment of the pipe; nearby equipment (for example, presence of a nearby road or airport that may create vibration that diminish the lifespan of the pipe); water quality; pressure; pressure variation; surge; traffic load; ground movement).

A condition assessment procedure designates any procedure to assess the condition of a pipe using a kind of inspection or analysis of the pipe. Non limitative examples of condition assessment procedures thus include visual inspection, measurements that may be performed on-site (acoustical analysis, electromagnetic analysis...) or be collecting samples an analyzing it in a laboratory. More generally, any kind of human inspection or measurements performed on the pipes that allows assessing the condition of the pipe can be used as a condition assessment procedure. A pipe condition score is a score that characterizes the condition of a given pipe section. A pipe condition score can be expressed using various scales. For example scales providing an indicative state of the pipe (such "good", "average", "poor"...), normalized scales between 0 and 1, or a measurements indicative of the pipe condition (such as pipe thickness) may be used.

The first step groups the pipe sections into a number of classes, and, within each class, the pipe sections are affected with a substantially similar degradation process.

As the condition assessment procedures of pipes are long and costly, the objective of the second step is to provide, for each class, a limited number of pipe sections that are as representative as possible of the pipe sections of the class, so that the condition assessment of a limited number of pipe sections in the class allows extracting general rules for estimating pipe conditions of pipe sections of the class based on pipe parameters. The condition assessment procedures provide, for the selected pipe sections, reliable pipe condition scores, because they rely on an inspection, observations or measurement performed on the actual pipe.

The fourth step consists in performing an estimation of pipe condition for the pipe sections for which no condition assessment procedure has been conducted. Since the pipe sections are grouped into classes of pipes affected by substantially similar degradation processes, and a condition assessment procedure has been conducted for some pipe sections within each class, the fourth step is accurately parameterized, and provides a reliable estimation of pipe condition.

The method of the invention thus allows an accurate estimation of pipe condition of pipes in a large pipe network, without requiring an exhaustive inspection of pipes, and thus with limited inspection costs.

The method of the invention reduces costs for estimating pipe condition, and planning pipe renewal.

The method of the invention allows efficient pipe inspection and renewal plans, and thus allows an efficient maintenance of the pipe network.

Advantageously, said number of classes is a predefined number of classes, and the first step comprises the application of a General Mixt Model (GMM) to the pipes for clustering the pipe sections into said predefined number of classes.

This allows grouping the pipe sections into classes having members with homogeneous features, thus allowing an efficient clustering of the pipe sections.

As indicated above, the second step of extracting the sample comprises: a first sub-step of initializing a set of candidate samples of pipe sections; a second sub-step of iteratively modifying said set of candidate samples using: a genetic algorithm based on an objective function comprising a minimization of the difference of average pipe parameters of the pipe sections of the sample, and the average pipe parameters of the pipe sections of the class; a third sub-step of selecting the candidate sample that optimizes said objective function.

The genetic algorithm optimizes, over successive iterations, the objective function that consists in minimizing the differences between average parameters of the sample and the class. The genetic algorithm provides the advantage of allowing an optimization the function while testing samples comprising very different combinations of pipe sections. This therefore allows obtaining samples whose average pipe parameters are as close as possible to the average pipe parameters of the corresponding classes. The samples thus provide a good representation of the classes, and reliable models of the classes can be built from these samples. Moreover, genetic algorithms have a limited computational complexity.

Advantageously, the relative size of each samples is negatively correlated with the homogeneity of each corresponding class.

In general, the more homogenous a class is, the lower the size of the sample needs to be to model accurately the degradation behavior of pipe sections of the class. Indeed, a lower number of training values are necessary to train a model, if the members of the class are homogeneous. Therefore, negatively correlating the relative sizes of the samples with the relative homogeneities of the classes allows allocating a size of samples to optimize the reliability of the predictions for all classes for a given number of pipe condition procedures to conduct.

Advantageously, the condition assessment procedure of the third step is chosen in a group comprising one or more of: an analysis of an electromagnetic flux applied to the pipe section; an acoustical analysis of the pipe section; the extraction, and analysis in a laboratory of a sample of the pipe section; and wherein each of the condition assessment procedure provides pipe condition scores at the same scale.

This allows selecting the most adapted measurement or observation method for each pipe section, and comparing easily the measurements or observations between the pipe sections.

Advantageously, the condition assessment procedures provide two or more pipe condition scores corresponding to different parts of pipe sections and chosen in a group comprising: an inner coating condition score; an outer coating condition score; a joint condition score.

This allows evaluating the evolution of the condition of different parts of pipe sections. This is useful, because the condition of different parts of the pipes can evolve in different ways depending on the structural and environmental pipe parameters.

Advantageously, a single pipe condition score is obtained from the two or more pipe condition scores corresponding to different parts of pipe sections, using a weighted or orthogonal sum.

This allows summarizing the pipe condition within a single pipe condition score that takes into account the specific degradations of different parts of the pipe.

Advantageously, the one or more pipe condition scores are associated with one or more reliability indexes defining the precision of the measurements that were used to obtain the one or more pipe condition scores.

This allows taking into account the reliability of each measurement in order to model as efficiently as possible the evolution of pipe conditions.

Advantageously, performing an estimation of one or more pipe condition scores for pipe sections that do not belong to the sample comprises: training, for a class, a supervised machine learning engine that predicts pipe condition scores based on pipe parameters using pipe sections that belongs to the sample; using said supervised machine learning engine to predict pipe condition scores based on pipe parameters of the pipe sections of the class that do not belong to the sample.

This allows an efficient modeling of the pipe conditions, because the pipes have been previously clustered into classes of similar pipe sections, and a model is trained for each class. The model is therefore well adapted to the pipe sections on which it will be applied.

Advantageously, said supervised machine learning engine is a random forest machine learning engine.

A random forest algorithm is especially well suited for this task. Indeed, a random forest removes the pipe parameters that are not predictive of pipe conditions. This is especially effective here, because a large number of structural or environmental parameters may be used. The random forest algorithm automatically uses only the parameters that actually allow predicting the pipe condition.

Advantageously, the method further comprises raising an alert for pipe sections whose pipe condition scores match an alert condition.

Alerts may be processed automatically, or displayed to the user in different forms (list, messages, maps...). This allows the operators to be aware of critical pipes, and triggering subsequent actions if necessary.

Advantageously, each alert for a pipe section automatically triggers at least one action chosen in a group comprising a further condition assessment procedure of the pipe section, a safeguard measure, and a repair of the pipe section.

Conditions assessment procedures allow accurately determining the actual condition of the pipe section. Thus, this allows using costly condition assessment procedures only for pipe sections that can be in a critical state. This ensures the safety of the pipe network at a reasonable cost by limiting the further condition assessment procedures to the pipes for which they are needed.

Safeguard measures may be any measure that may avoid causing damages due to a broken pipe section, such as closing the valves to isolate the pipe section. This allows avoiding the creation of damages by a broken pipe until actual pipe condition is assessed and/or the pipe section is repaired.

The invention also discloses computer program product, stored on a non-transitory computer-readable medium, said computer program product comprising code instructions for executing a method according to an aspect of the invention.

The invention also discloses a device comprising a processor configured to execute a method according to an aspect of the invention.

### BRIEF DESCRIPTION OF THE DRAWINGS

The invention will be better understood and its various characteristics and advantages will emerge from the following description of a number of exemplary embodiments and its appended figures in which:
- Figure 1 displays a network of pipes on which a method according to the invention may be implemented;
- Figure 2 displays a computer-implemented method according to the invention;
- Figure 3 displays an example of clustering of pipes in an embodiment of the invention;
- Figure 4 displays a graph showing the accuracy of a method in an embodiment of the invention depending upon the relative size of the sample, and the material of a pipe;
- Figure 5 displays a pipe inspection plan of an example of computer-implemented method in an embodiment of the invention.

### DETAILED DESCRIPTION OF THE INVENTION

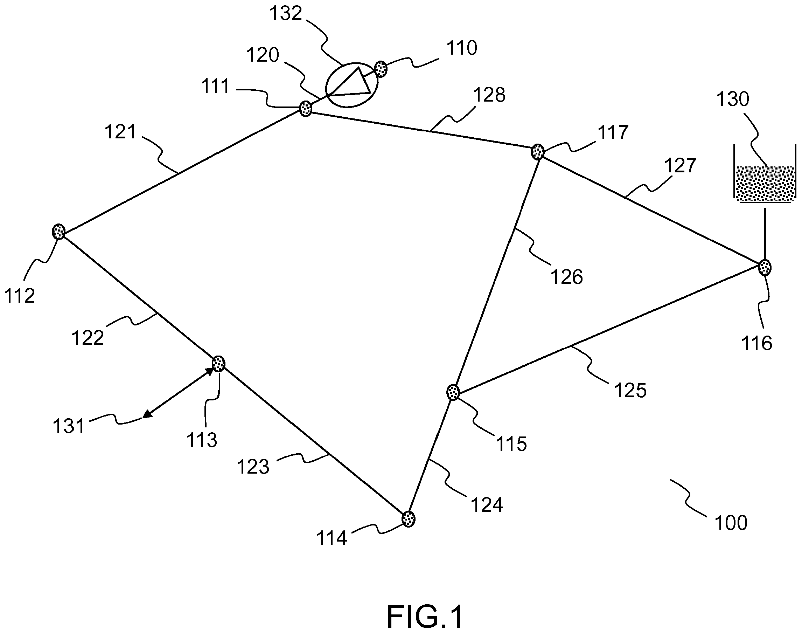

Figure 1 displays a network of pipes on which a method according to the invention may be implemented.

The network 100 displayed on figure 1 represents a water distribution network and comprises a plurality of nodes 110, 111, 112, 113, 114, 115, 116 and 117, and a plurality of arcs 120, 121, 122, 123, 124, 125, 126, 127 and 128. The nodes typically represent the connections with water sources or water reservoirs, for example the reservoir 130 at node 116, the connections with the user of the water distribution system, for example consumption 131 at node 113 and the connections between the arcs. The arcs represent pipes between the nodes. The network can be equipped with equipment such as valves and pumps. A pump 132 is for example present on the arc 120. More generally, a node can be a junction between two or three pipes, a point at which inputs or outputs of the network are found, for example a point where a user consumes water, or a point at which water is injected in the network. A node can also represent a sub-network, for example a neighborhood grouped under a single node.

The exemplary network of figure 1 has a low number of pipes. However, typical water network has a much higher number of pipes. For example, the invention may be applied to pipe networks comprising 10,000, or even more, km of pipes. For example, the invention can be applied to pipe networks representing a metropolitan area. For example, the drinking water distribution network of Paris area comprises around 37,000 km of pipes; the drinking water distribution network of Tokyo area comprises around 27,000 km of pipes; the drinking water distribution network of London area comprises around 21,000 km of pipes; the drinking water distribution network of Adelaïde area comprises around 9,000 km of pipes; the drinking water distribution network of Casablanca area comprises 4,500 km of pipes. The total length of drinking water pipes in the European Union is estimated to be around 4,230,000 km.

Each pipe of the network may be progressively degraded over time. An objective of the invention is thus to obtain a reliable estimation of pipe condition in order to plan pipe inspection, repair and renewal and as efficiently as possible.

Although figure 1 displays a water distribution network, the invention may be applied to other kinds of fluid networks, such as oil or gas distribution networks.

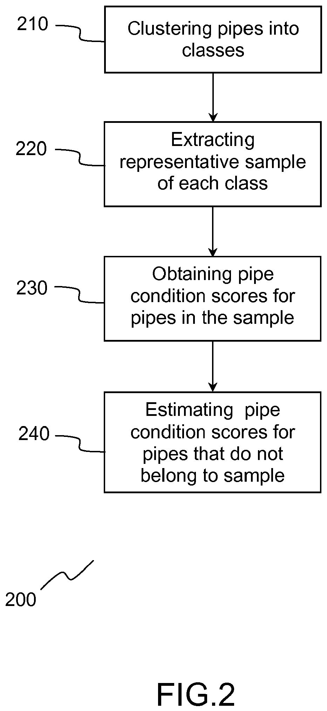

The figure 2 displays a computer-implemented method according to the invention.

The method 200 applies to a pipe network, that may be for example a water, oil or gas distribution network. The network is formed of interconnected pipes. The pipes are split into sections. A pipe section may be either an entire pipe, or a part of a pipe. It may be useful to split large pipes into a plurality of sections, because the section may be located into different environments, and thus pipe condition may evolve differently over time for the various sections of the same pipe. Conversely, adjacent and interconnected pipes may be grouped within the same pipe section, if they share certain properties (for example, the same material, thickness, etc...). Pipe sections may be defined according to different rules. For example, the pipes may be split into sections:
- according to changes in features of the pipes or environment changes (i.e, a pipe can be split in sections if the material of the pipe changes, if there is a significant modification of the environment over the length of the pie, etc...);
- graphically, by a user defining manually the limits of pipe sections;
- to reflect a maximum length of a single pipe section.

The method 200 can be implemented within an asset management solution that allows controlling the condition of pipes in a network, and planning pipe maintenance and renewal. Such a solution may also allow the management of other assets of the network, such as reservoir, pumps, etc...

An asset management solution has access to one or more data storages that store information relative to the assets. For example, this may grant access to values of a number of pipe parameters. Such pipe parameters may for example comprise one or more of:
- structural parameters:
   - length of the pipe;
   - material of the pipe;
   - diameter of the pipe;
   - age of the pipe;
- environmental parameters:
   - temperatures of the environment of the pipe;
   - humidity of the environment of the pipe;
   - nearby equipment (for example, presence of a nearby road or airport that may create vibration that diminish the lifespan of the pipe);
   - water quality;
   - pressure;
   - pressure variation;
   - surge;
   - traffic load;
   - ground movement;

These parameters have an influence on the evolution of pipe condition, and the probability of pipe failure over time. For example, a pipe that is subject to extreme temperature, high humidity, or the vibration created by a nearby infrastructure will be subject to a faster degradation. The degradation rate also depends on structural parameters of the pipes, for example the material and diameter of the pipe. Certain parameters may also interact. For example, the impact of humidity on the pipe may be dependent upon the material of the pipe.

In a number of embodiments of the invention, the values of pipe parameters can be retrieved, for each pipe section, using some kind of storage or database.

The method 200 comprises a first step 210 of clustering pipe sections of a pipe network into a number of classes, based on pipe parameters.

This first step 210 consists in grouping the pipe sections that share similar parameters within the same class. The pipe sections that belong to the same class are thus likely to evolve in a similar way over time.

This can be performed in different ways. Unsupervised machine learning algorithms are especially well suited for clustering the pipes. Clustering can be performed based on any combination of pipe parameters. For example, clustering can be performed according to one or more parameters expected to have an impact on pipe degradation: material, diameter, length, pressure, water quality, ground quality, stay current, road traffic, etc... Clustering can be performed to split the pipe sections into a target number of classes, for example a target number of classes predefined by an operator. For example, a target number of four classes can be used. The number of classes can also be adapted to avoid having classes with a very low number of members, which may not be very significant. The number of classes can also be adapted to the size of the network: for example, a lower number of classes can be used for small networks (i.e, networks having a total length of pipes equal to or lower than a predefined threshold). Classes can also be merged a posteriori. For example, a class with a low number of members can be merged with a class having similar features.

Different models or algorithms can be used to perform the clustering. For example, a General Mixt Model (GMM) model provides good clustering results, because it allows obtaining classes having homogenous members.

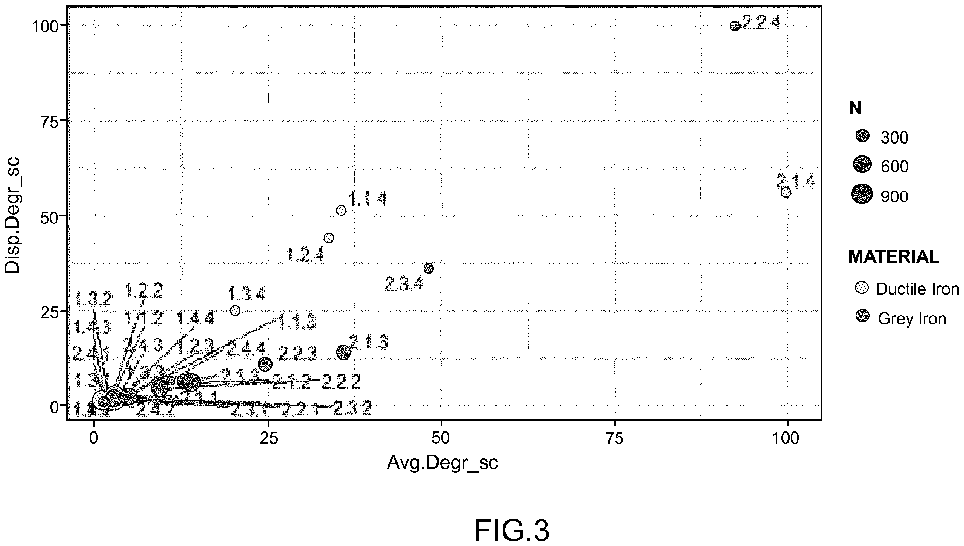

Figure 3 displays an example of clustering of pipes in an embodiment of the invention.

The pipes have been clustered according to a plurality of variables including pipe material and diameter.

The table below show some characteristics of each of the clusters represented in figure 3 (number of pipes, total length of the pipes, and average degradation indicator of the pipes). The average degradation indicator is, in this example, a value between 0 and 1 that indicates the average degradation of pipes in the cluster. It is calculated based on the pipes that have been inspected, and the higher it is, the more degraded pipes are.

**Table 1 - Statistics relative to clusters of pipes**

| ***Cluster*** | ***Number of pipe*** | ***Total Length (km)*** | ***Average Degradation Indicator*** |
|---|---|---|---|
| 1.1.1 | 1020 | 13,0638 | 0,00073396 |
| 1.1.2 | 2258 | 135,3849 | 0,0024462 |
| 1.1.3 | 815 | 120,05081 | 0,0064775 |
| 1.1.4 | 166 | 27,93472 | 0,05668984 |
| 1.2.1 | 530 | 7,21911 | 0,00052549 |
| 1.2.2 | 789 | 62,2254 | 0,00194201 |
| 1.2.3 | 284 | 59,06881 | 0,00553672 |
| 1.2.4 | 47 | 10,98917 | 0,03270694 |
| 1.3.1 | 382 | 28,36028 | 0,00056146 |
| 1.3.2 | 80 | 23,96238 | 0,00243233 |
| 2.1.1 | 782 | 9,87718 | 0,00612382 |
| 2.1.2 | 1254 | 86,93611 | 0,02006067 |
| 2.1.3 | 510 | 73,66109 | 0,05917312 |
| 2.1.4 | 148 | 31,74353 | 0,18194432 |
| 2.2.1 | 668 | 17,24092 | 0,00573948 |
| 2.2.2 | 306 | 42,75907 | 0,02044657 |
| 2.2.3 | 83 | 27,5024 | 0,0816995 |
| 2.3.1 | 251 | 11,7555 | 0,00253111 |
| 2.3.2 | 89 | 17,10164 | 0,00913077 |
| 2.3.3 | 33 | 12,83664 | 0,02550907 |
| 3.1.1 | 469 | 24,27088 | 0,00239257 |
| 4.1.1 | 50 | 9,86144 | 0,0112026 |

Coming back to figure 2, the method 200 comprises a second step 220 of extracting, for each class, a sample of pipe sections of the class.

This step consists in identifying, for each class, a number of pipe sections to inspect. As will be explained in more details hereinafter, these identified pipe sections will be inspected on-site to obtain a pipe condition score, in order to be able to estimate pipe condition scores from pipe parameters within each class. The second step 220 provides, for each class, a limited number of pipe sections that are as representative as possible of the pipe sections of the class.

The pipe sections of the sample may be selected according to a number of techniques and constraints.

According to various embodiments of the invention, the sample can be set to comprise a maximum number or percentage of pipe sections of the class. For example, the sample may comprise twenty pipe sections among a hundred pipe sections that belong to a class. This maximum number or percentage may be defined in different ways. For example, it may be defined by an operator, because it corresponds to a number of pipes that is expected to be sufficient to allow a good estimation of the pipe condition scores, and/or due a maximum number of pipe sections that matches a defined budget for pipe inspection.

In a number of embodiments of the invention, a total budget for pipe inspection, corresponding to a defined number of pipe condition assessment procedures, is allocated among the different classes. This number of pipe conditions procedures can be split between the different samples. The sizes of the samples can be defined according to the relative sizes and homogeneity of the classes. Indeed, a lesser number of pipe inspection procedures are required to provide a reliable modeling of homogeneous class. Thus, a lower size of the sample can be allocated to more homogeneous classes.

The applicant has filed, in the domain of real-time estimation of fluid consumption, an international patent application published under n° WO 2014/060655. The method of estimation of fluid consumption relies on the classification of users into classes of users having similar consumption profiles, the real time measurement of consumption of a sample of users for each class, and the estimation a total consumption of the network based on these partial real time measurements.

This patent application faces the same problem allocating an optimal number of users to different classes in order to have the best global estimation with a limited number of real time measurements. In order to solve this issue, the applicant has defined a formula to define the sizes of the samples depending of the total target size of the samples, the size and dispersion of each class. This formula is provided p. 7 I. 15-28 of the said international publication. This formula can be applied mutadis mutandis to the allocation of the sizes of the samples of pipe sections of the invention, based on the relative sizes and dispersion of values of pipe parameters in the invention. The dispersion may here be the dispersion of the pipe conditions scores, or more generally a degradation indicator of each pipe that may be calculated based on past failures, or the same parameters than the pipe condition scores. Here, the sizes of the samples allows an efficient modeling of each class, because the classes with more heterogeneous pipes will be modeled using a relatively higher number of pipe condition assessments.

In a number of embodiments of the invention, the sample comprises pipe sections, for which an inspection has been previously planned. This may be for example pipe sections that are already expected to be already in a critical state, and/or that are planned to be inspected by the inspection plan of the operator. This allows saving pipe inspection budget, because the inspection that are already planned will be used to feed the sample.

In an example not according to the invention, a sample comprises randomly selected pipe sections. This provides the advantage of being simple, but fails to ensure that the selected pipe sections are fairly representative of the class.

According to the invention, a sample comprises pipe sections that are expected to be the most representative of the class. This allows obtaining a sample that provides, for a given number of pipes, the best estimation of pipe condition based on pipe parameters. The pipe sections can be expected to be the most representative of the class, for example if their pipe parameters are as close as possible to the average of pipe parameters within the class. As an example not according to the invention, this can be performed for each pipe section individually, by ensuring that the parameters of each selected pipe section are individually as close as possible to the average of the pipe sections of the class.

It can also be performed for the sample as a whole, by ensuring that the average of the parameters of the pipes of the sample are as close as possible to the average of the parameters of the pipes. This presents the advantage of ensuring that the pipe sections of the sample are, as a whole, representative of the pipe sections of the class. It also allows selecting a sample of pipe sections representative of the class, even if the selection some pipe sections of the samples is constrained (for example because some pipe sections are mandatorily selected because they belong to the pipe inspection plan of the operator). According to the invention, the pipe sections of the sample are selected using a genetic algorithm. This consists in initializing a set of candidate samples, then iteratively modifying this set using selection, crossover and mutation, in order to iteratively obtain better candidate samples.

Genetic algorithms use an objective function that allows ranking candidate samples in order to select the best candidates. According to the invention, the objective function comprises a minimization of a difference between the average of pipe parameters of the sample, and the average of pipe parameters of the class. Thus, over successive iterations, the candidate pipe samples become more representative of the class.

According to various embodiments of the invention, all the pipe sections of the sample can be modified at each iteration, or some pipe sections are kept in all samples (for example, pipe sections that will be inspected anyway in the pipe inspection plan of the operator). The genetic algorithm provides the ability of obtaining sample of pipes that are representative of the class, even with constraints relative to the selection of pipe sections.

Finally, the candidate sample that optimizes the cost function, that is to say the candidate sample that is considered as the most representative of the class, is selected.

The method 200 further comprises a third step 230 of obtaining, for each pipe section in each sample for each class, one or more pipe condition scores determined by a condition assessment procedure.

As explained above, the pipe sections that are selected for being part of a sample are inspected. Different condition assessment procedures may be used. For example, the pipe conditions may be assessed using:
- an electromagnetic flux;
- an acoustical analysis;
- the extraction of sample of the pipes, that are being analyzed in a laboratory;
- visual inspection;
- CCTV and computer vision;
- Tomography;
- Drone inspection;

The condition assessment procedure may be the same for all the pipe sections, or may be selected specifically for each pipe section. The selection of a condition assessment procedure may be performed, depending on the technical feasibility of each method in the environment of the pipe section, or because a procedure is particularly well suited for a given pipe section. For example, metallic pipes can be inspected with electromagnetic flux or acoustic techniques, while non-metallic pipes can be inspected by collecting a sample and sending it to a laboratory. The inspection technique can also be chosen according to the criticality of a pipe, defined by the operator. For example, a pipe section that serves an hospital, or a city center, can be considered as more critical. Thus a more accurate pipe condition assessment procedure, or a procedure that provides more information about the pipe, such as sample collection and laboratory analysis, can be chosen, even if it is more expensive.

The pipe inspection procedure can also be chosen according to its technical feasibility. For example, acoustical inspection is an inexpensive method that does not require to open a trench to reach the pipe, but current acoustical inspection can be performed only on metallic pipes of less than two hundred meters long. An acoustical inspection can thus be performed each time it is technically possible, and if it is not, other methods which are more expensive may be used.

Each of these condition assessment procedures allows providing one or more pipe condition scores of the pipe section that has been inspected. The pipe condition score indicates a level of degradation of the pipe section. When different procedures are used, their output may be expressed using different scales. For example, an acoustical analysis provides a residual thickness of a pipe (or, conversely, a value or percentage of lost thickness), while a laboratory test of a sample of a pipe provides a number of measurements of the pipe such as a corrosion presence and type of corrosion for internal and/or external corrosion, residual thickness, obstruction level, a graphitization level, etc...

In order to obtain homogeneous and comparable measurements the pipe condition scores can be transformed to a normalized scale, so that different inspection methods provide a pipe condition score using the same scale, and all the pipe conditions scores can be compared. For example, pipe condition scores ranging from 0 to 1 can be used, wherein 0 corresponds to a perfect condition, and 1 a failure condition. Thus, each pipe section can be inspected using the most relevant procedure (depending on pipe section characteristics and/or the feasibility of different inspection methods), while the output of the procedures can be compared. For example, if an acoustical analysis is used, the residual thicknesses can be transformed into homogeneous values between 0 and 1; in laboratory tests are used, scores relative to the different analysis parameters can be weighted according to their relative importance.

The pipe condition scores may also belong to a finite number of states, for example 3 states: "good", "average" or "poor".

In a number of embodiments of the invention, a plurality of condition scores are obtained for each pipe, which correspond to different parts of the pipe section. For example, the observation can generate a vector of 3 pipe conditions scores for a pipe section:
- an inner coating condition score;
- an outer coating condition score;
- a joint condition score.

This allows evaluating the evolution of the condition of different parts of pipe sections. This is useful, because the condition of different parts of the pipes can evolve in different ways depending on the structural and environmental pipe parameters. Using a vector of pipe condition scores that correspond to various parts of the pipe section therefore allows taking into account the degradation of different parts of the pipe.

The pipe condition scores or the vector can then be summed using an orthogonal or weighted sum, in order to obtain a single pipe condition score for the pipe section. This allows summarizing the pipe condition within a single pipe condition score that takes into account the specific degradations of different parts of the pipe.

In a number of embodiments of the invention, the pipe condition scores are associated with one or more reliability indexes. The reliability indexes indicate how precise and reliable the observed pipe condition scores are. For example, the reliability indexes may depend upon the observation method. For example, it can depend upon the reliability of the instrument that performed the measure. A measure using instrument can also be considered as more reliable than visual observation.

According to a number of embodiments of the invention, when a vector of a plurality of pipe condition scores corresponding to different parts of a pipe section is obtained, there may be either a single reliability index for the whole vector, or a reliability index for each pipe condition in the vector. Using a reliability index for each pipe condition in the vector allow tailoring the indication of reliability of the measures or observations, when the accuracy of an observation methods is variable for the different parts of the pipe. For example, a visual observation is expected to be more reliable for evaluating the condition of the outer coating than the condition of inner coating of the pipe. Pipe condition scores resulting from visual observations can thus be associated with average reliability index for outer coating, but low reliability index for inner coating.

The method 200 further comprises a fourth step 240 of performing an estimation of one or more pipe condition scores for pipe sections that do not belong to a sample based on said pipe parameters, said estimation being parameterized with the pipe condition scores and pipe parameters for the pipe sections in the samples.

This step consists in training a model, for each of the classes, based on the pipe condition scores that are obtained through pipe condition assessment procedures, then using the model for estimating pipe conditions scores of pipes that were not subject of a condition assessment procedure.

This process allows an efficient modeling of the pipe conditions, because the pipes have been previously clustered into classes of similar pipe sections, and a model is trained for each class. The model is therefore well adapted to the pipe sections on which it will be applied.

This step may be performed in different ways. For example, a statistical model of linear regression can be trained, for each class, by fitting the pipe parameters to the pipe condition scores, for the pipe sections whose conditions have been assessed.

This can also be achieved by training a supervised machine learning model with the pipes whose conditions have been assessed. Thus, a machine learning engine can be fed, for each class, with the pipe parameters and pipe condition scores of the pipes sections that have been inspected, then used to predict pipe condition scores based on pipe parameters for the pipe sections that have not been inspected.

A random forest algorithm is especially well suited for this task. Indeed, a random forest removes the pipe parameters that are not predictive of pipe conditions. This is especially effective here, because a large number of structural or environmental parameters may be used. The random forest algorithm automatically uses only the parameters that actually allow predicting the pipe condition.

The method 200 therefore allows an accurate estimation of the state of each pipe in a large pipe network, while performing inspection of only a limited number of pipes, which allows saving money and time.

The estimations of pipe conditions can then be used to improve the management of the pipe network, in different ways.

For example, the pipe conditions of pipe sections can be displayed to an operator, so that the operator is able to determine which are the pipe sections to inspect in priority. This allows adapting pipe inspection plans. For example, the pipes that are expected to be in the most critical state can be inspected in priority. The determination of the order in which pipes should be inspected can be performed either manually or by an operator. This may also be performed by displaying a map with colors corresponding to the expected state of the pipes. For example, the pipes that are expected to be in a good state can be displayed in green, while pipes that are expected to be in a degraded state can be displayed in red. This also allows obtaining, in general, an estimation of the state of the pipe network.

An alert can also be generated for pipes that are expected to be in a critical or more generally a degraded state. An alert can for example be generated for pipe sections whose pipe condition score match an alert condition. The alert condition may typically be a predefined pipe condition score threshold, all pipes whose condition score is below the threshold raising an alert. The alert condition may also be a combination of condition score and other parameters. For example, the score threshold under which an alert is generated may depend upon the material of the pipe. Alternatively, a fixed percentage of the pie sections having the lowest scores may trigger an alert.

Once the alert is generated, the alert and information relative to the pipe can be displayed. The display of the alert may take different forms: display of a list of critical pipes, display of a map comprising a color codes for the pipes (e.g critical pipes in red, and non-critical pipes in green).

Each alert may also trigger a costlier and more reliable inspection (sending a drone, a smart ball that inspects the inside of a canalization ...). Thus, pipes expected to be critical benefit from an even more reliable inspection to determine the actual state of the pipe and decide if repair of the pipe is needed.

Safeguard measures can be executed while waiting for the result of this additional inspection and/or fix of the pipe. For example, valves can be closed in order to separate the pipe from the rest of the network until the pipe is further analyzed and fixed. Pipes for which an alert have been raised may be fixed automatically, for example by sending autonomous drones to repair the pipes.

As this is performed only on some pipe sections that are expected to be in a critical state, the method of the invention provides safety to the network while not being expensive.

At each state of the method, the data that is obtained may be presented to an operator for validation. This allows ensuring that no incoherent value is used, and identify potential discrepancies in the data relative to the pipe network.

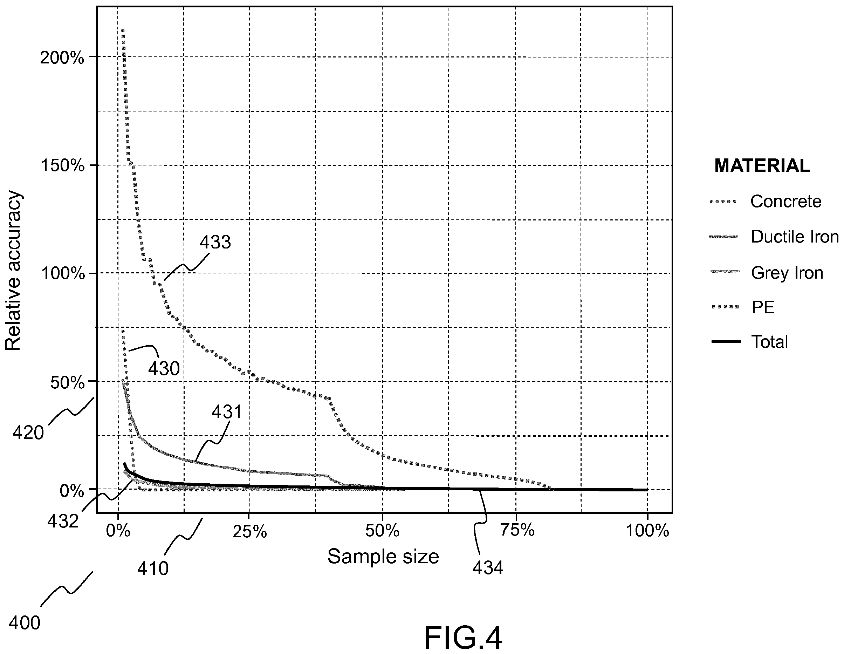

Figure 4 displays a graph showing the accuracy of a method in an embodiment of the invention depending upon the relative size of the sample, and the material of a pipe.

In the graph 400:
- the horizontal axis 410 represents the relative size of a sample, that is to say the percentage of pipes of a class that belong to a sample and that will be inspected;
- the vertical axis 420 represents the relative accuracy of the estimation of pipe condition. This relative accuracy corresponds to an error, expressed as a percentage of an expected error that depends on the size of the sample;
- the curves 430, 431, 432, 433, 434 represent the evolution of relative accuracy as a function of sample size, respectively for pipes made of concrete, ductile iron, grey iron, PE (PolyEthylen), and all the pipes.

This example demonstrates that, the higher the size of the sample of pipe sections to inspect relative to a class, the higher the accuracy of the estimation. However, the actual value of accuracy depending on sample size depends upon pipe material. For example, a relative accuracy of 10% or less is achieved with a relative sample size around 3-4% for ductile iron pipes, but around 45% for PE pipes. Therefore, it is possible to select, depending upon the pipe material, a sample size which is just sufficient to achieve a desired accuracy. This allows obtaining a target accuracy while limiting the number of inspection depending upon the pipe material.

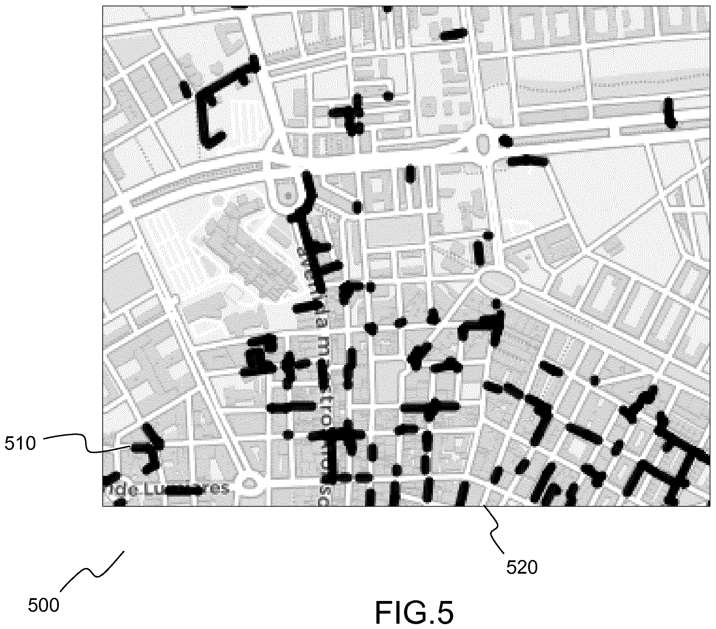

Figure 5 displays a pipe map at an output of an example of computer-implemented method in an embodiment of the invention.

The plan 500 comprises represents a map of pipes. The locations of the pipe that are expected to be degraded are represented by grey circles, such as the circle 510 and 520.

The pipe inspection plan 500 represents all pipe sections of a pipe network that have been selected for inspection by a method according to the invention. This therefore allows operators to plan inspection, for example by inspecting in the same time nearby pipe sections.

The examples described above are given as illustrations of embodiments of the invention, and demonstrate the ability of the invention to provide a reliable estimation of pipe condition of pipes of a network when inspecting only a subset of the pipes, and thus at a moderate cost. They do not in any way limit the scope of the invention which is defined by the following claims.

## Claims

1. A computer-implemented method (200) comprising:
- a first step of clustering (210) pipe sections of a pipe network into a number of classes, based on pipe parameters relative to the structure or to the environment of the pipe sections;
- and, for each class of said number of classes:
- a second step of extracting (220) a sample of pipe sections of the class, said second step comprising:
▪ a first sub-step of initializing a set of candidate samples of pipe sections;
▪ a second sub-step of iteratively modifying said set of candidate samples using:
• a genetic algorithm based on an objective function comprising a minimization of the difference of average pipe parameters of the pipe sections of the sample, and
• the average pipe parameters of the pipe sections of the class;
▪ a third sub-step of selecting the candidate sample that optimizes said objective function ;
- a third step of obtaining (230), for each pipe section sample, one or more pipe condition scores determined by a condition assessment procedure;
- a fourth step of performing (240) an estimation of one or more pipe condition scores for pipe sections that do not belong to the sample based on said pipe parameters, said estimation being parameterized with the pipe condition scores and pipe parameters of the pipe sections of the sample extracted at the second step.

2. The computer-implemented method of claim 1, wherein said number of classes is a predefined number of classes.

3. The computer-implemented method according to any one of claims 1 or 2, wherein the relative size of each sample is negatively correlated with the homogeneity of each corresponding class.

4. The computer-implemented method of one of claims 1 to 3, wherein the condition assessment procedure of the third step is chosen in a group comprising one or more of:
- an analysis of an electromagnetic flux applied to the pipe section;
- an acoustical analysis of the pipe section;
- the extraction, and analysis in a laboratory of a sample of the pipe section;
and wherein each of the condition assessment procedure provides pipe condition scores at the same scale.

5. The computer-implemented method of claim 4 wherein the condition assessment procedures provide two or more pipe condition scores corresponding to different parts of pipe sections and chosen in a group comprising:
- an inner coating condition score;
- an outer coating condition score;
- a joint condition score.

6. The computer-implemented method of claim 5, wherein a single pipe condition score is obtained from the two or more pipe condition scores corresponding to different parts of pipe sections, using a weighted or orthogonal sum.

7. The computer-implemented method of one of claims 4 to 6, wherein the one or more pipe condition scores are associated with one or more reliability indexes defining the precision of the measurements that were used to obtain the one or more pipe condition scores.

8. The computer-implemented method of one of claims 1 to 7, wherein performing an estimation of one or more pipe condition scores for pipe sections that do not belong to the sample comprises:
- training, for a class, a supervised machine learning engine that predicts pipe condition scores based on pipe parameters using pipe sections that belongs to the sample;
- using said supervised machine learning engine to predict pipe condition scores based on pipe parameters of the pipe sections of the class that do not belong to the sample.

9. The computer-implemented method of claim 8, wherein said supervised machine learning engine is a random forest machine learning engine.

10. The computer-implemented method of claim 1 to 9, further comprising raising an alert for pipe sections whose pipe condition scores match an alert condition.

11. The computer-implemented method of claim 10, wherein each alert for a pipe section automatically triggers at least one action chosen in a group comprising a further condition assessment procedure of the pipe section, a safeguard measure, and a repair of the pipe section.

12. A computer program product, stored on a non-transitory computer-readable medium, said computer program product comprising code instructions which, when the program is executed by a computer, cause the computer to carry out the method according to any one of claims 1 to 11.

13. A data processing device comprising a processor configured to execute a method according to any one of claims 1 to 11.

## Patentansprüche

1. Computerimplementiertes Verfahren (200), umfassend:
- einen ersten Schritt des Bündelns (210) von Rohrabschnitten eines Rohrnetzwerks in eine Anzahl von Klassen auf der Grundlage von Rohrparametern in Bezug auf die Struktur oder auf die Umgebung der Rohrabschnitte;
und für jede Klasse der Anzahl von Klassen:
- einen zweiten Schritt des Extrahierens (220) einer Stichprobe von Rohrabschnitten der Klasse, wobei der zweite Schritt umfasst:
- einen ersten Teilschritt des Initialisierens einer Menge von Stichprobenkandidaten von Rohrabschnitten;
- einen zweiten Teilschritt des iterativen Modifizierens der Menge von Stichprobenkandidaten unter Verwendung von Folgendem:
- ein genetischer Algorithmus, der auf einer Zielfunktion beruht, die eine Minimierung der Differenz durchschnittlicher Rohrparameter der Rohrabschnitte der Stichprobe umfasst, und
- die durchschnittlichen Rohrparameter der Rohrabschnitte der Klasse;
- einen dritten Teilschritt des Auswählens desjenigen Stichprobenkandidaten, der die Zielfunktion optimiert;
- einen dritten Schritt des Erlangens (230) einer oder mehrerer Rohrzustandsbewertungen, die durch eine Zustandsbeurteilungsprozedur bestimmt werden, für jede Rohrabschnitt-Stichprobe;
- einen vierten Schritt des Durchführens (240) einer Schätzung einer oder mehrerer Rohrzustandsbewertungen für Rohrabschnitte, die nicht zur Stichprobe gehören, auf der Grundlage der Rohrparameter, wobei die Schätzung mit den Rohrzustandsbewertungen und Rohrparametern der Rohrabschnitte der im zweiten Schritt extrahierten Stichprobe parametrisiert wird.

2. Computerimplementiertes Verfahren nach Anspruch 1, worin die Anzahl von Klassen eine vordefinierte Anzahl von Klassen ist.

3. Computerimplementiertes Verfahren nach einem der Ansprüche 1 oder 2, worin die relative Größe jeder Stichprobe negativ mit der Homogenität jeder entsprechenden Klasse korreliert ist.

4. Computerimplementiertes Verfahren nach einem der Ansprüche 1 bis 3, worin die Zustandsbeurteilungsprozedur des dritten Schritts in einer Gruppe gewählt wird, die eines oder mehr von Folgendem umfasst:
- eine Analyse eines elektromagnetischen Flusses, der an den Rohrabschnitt angelegt wird;
- eine akustische Analyse des Rohrabschnitts;
- die Entnahme einer Probe des Rohrabschnitts und Analyse in einem Labor;
und worin jede der Zustandsbeurteilungsprozeduren Rohrzustandsbewertungen im gleichen Maßstab bereitstellt.

5. Computerimplementiertes Verfahren nach Anspruch 4, worin die Zustandsbeurteilungsprozedur zwei oder mehr Rohrzustandsbewertungen bereitstellt, die unterschiedlichen Teilen von Rohrabschnitten entsprechen und in einer Gruppe gewählt werden, die Folgendes umfasst:
- eine Bewertung des Zustands der Innenbeschichtung;
- eine Bewertung des Zustands der Außenbeschichtung;
- eine Bewertung des Zustands der Verbindungsstellen.

6. Computerimplementiertes Verfahren nach Anspruch 5, worin aus den zwei oder mehr Rohrzustandsbewertungen, die unterschiedlichen Teilen von Rohrabschnitten entsprechen, unter Verwendung einer gewichteten oder orthogonalen Summe eine einzige Rohrzustandsbewertung erlangt wird.

7. Computerimplementiertes Verfahren nach einem der Ansprüche 4 bis 6, worin der einen oder den mehreren Rohrzustandsbewertungen ein oder mehrere Zuverlässigkeitsindizes zugeordnet werden, welche die Genauigkeit der Messungen definieren, die verwendet wurden, um die eine oder mehreren Rohrzustandsbewertungen zu erlangen.

8. Computerimplementiertes Verfahren nach einem der Ansprüche 1 bis 7, worin das Durchführen einer Schätzung einer oder mehrerer Rohrzustandsbewertungen für Rohrabschnitte, die nicht zur Stichprobe gehören, Folgendes umfasst:
- für eine Klasse erfolgendes Trainieren einer beaufsichtigten Maschinenlernen-Funktionseinheit, die Rohrzustandsbewertungen auf der Grundlage von Rohrparametern unter Verwendung von Rohrabschnitten, die zur Stichprobe gehören, vorhersagt;
- Verwenden der beaufsichtigten Maschinenlernen-Funktionseinheit, um Rohrzustandsbewertungen auf der Grundlage von Rohrparametern derjenigen Rohrabschnitte der Klasse, die nicht zur Stichprobe gehören, vorherzusagen.

9. Computerimplementiertes Verfahren nach Anspruch 8, worin die beaufsichtigte Maschinenlernen-Funktionseinheit eine Maschinenlernen-Funktionseinheit nach dem Random-Forest-Prinzip ist.

10. Computerimplementiertes Verfahren nach einem der Ansprüche 1 bis 9, ferner umfassend: Auslösen eines Alarms für Rohrabschnitte, deren Rohrzustandsbewertungen einer Alarmbedingung entsprechen.

11. Computerimplementiertes Verfahren nach Anspruch 10, worin jeder Alarm für einen Rohrabschnitt automatisch mindestens eine Aktion auslöst, die in einer Gruppe gewählt wird, die eine weitere Zustandsbeurteilungsprozedur des Rohrabschnitts, eine Schutzmaßnahme und eine Reparatur des Rohrabschnitts umfasst.

12. Computerprogrammprodukt, gespeichert auf einem nichtflüchtigen computerlesbaren Medium, wobei das Computerprogrammprodukt Codeanweisungen umfasst, die, wenn das Programm durch einen Computer ausgeführt wird, den Computer veranlassen, das Verfahren nach einem der Ansprüche 1 bis 11 auszuführen.

13. Datenverarbeitungsvorrichtung, umfassend einen Prozessor, der dafür konfiguriert ist, ein Verfahren nach einem der Ansprüche 1 bis 11 auszuführen.

## Revendications

1. Procédé mis en œuvre par ordinateur (200), comprenant :
- une première étape de groupement (210) de sections de canalisation d'un réseau de canalisations en un certain nombre de catégories, sur la base de paramètres de canalisation relatifs à la structure ou à l'environnement des sections de canalisation,
- et, pour chaque catégorie du dit nombre de catégories :
- une deuxième étape d'extraction (220) d'un échantillon de sections de canalisation de la catégorie, ladite deuxième étape comprenant :
- une première sous-étape d'initialisation d'un ensemble d'échantillons candidats de sections de canalisation,
- une deuxième sous-étape de modification de façon itérative dudit ensemble d'échantillons candidats en utilisant :
- un algorithme génétique basé sur une fonction cible comprenant une minimisation de la différence des paramètres de canalisation moyens des sections de canalisation de l'échantillon, et
- les paramètres de canalisation moyens des sections de canalisation de la catégorie, et
- une troisième sous-étape de sélection de l'échantillon candidat qui optimise ladite fonction cible,
- une troisième étape d'obtention (230), pour chaque échantillon de section de canalisation, d'un ou plusieurs scores d'état de canalisation déterminés par une procédure d'évaluation d'état,
- une quatrième étape d'exécution (240) d'une estimation d'un ou plusieurs scores d'état de canalisation pour des sections de canalisation qui ne font pas partie de l'échantillon sur la base des dits paramètres de canalisation, ladite estimation étant paramétrée avec les scores d'état de canalisation et des paramètres de canalisation des sections de canalisation de l'échantillon extrait à la deuxième étape.

2. Procédé mis en œuvre par ordinateur selon la revendication 1, dans lequel ledit nombre de catégories est un nombre prédéterminé de catégories.

3. Procédé mis en œuvre par ordinateur selon l'une quelconque des revendications 1 ou 2, dans lequel la taille relative de chaque échantillon est corrélée négativement avec l'homogénéité de chaque catégorie correspondante.

4. Procédé mis en œuvre par ordinateur selon l'une des revendications 1 à 3, dans lequel la procédure d'évaluation d'état de la troisième étape est sélectionnée dans un groupe comprenant une ou plusieurs des suivantes :
- une analyse d'un flux électromagnétique appliqué sur la section de canalisation,
- une analyse acoustique de la section de canalisation,
- l'extraction et une analyse dans un laboratoire d'un échantillon de la section de canalisation,
et dans lequel chaque procédure d'évaluation d'état fournit des scores d'état de canalisation à la même échelle.

5. Procédé mis en œuvre par ordinateur selon la revendication 4, dans lequel la procédure d'évaluation d'état fournit deux ou trois scores d'état de canalisation correspondant à différentes parties de sections de canalisation et sélectionnés dans un groupe comprenant :
- un score d'état de revêtement intérieur,
- un score d'état de revêtement extérieur,
- un score d'état de raccord.

6. Procédé mis en œuvre par ordinateur selon la revendication 5, dans lequel un score d'état de canalisation unique est obtenu à partir des deux scores d'état de canalisation ou plus correspondant à différentes parties des sections de canalisation, en utilisant une somme pondérée ou orthogonale.

7. Procédé mis en œuvre par ordinateur selon l'une des revendications 4 à 6, dans lequel l'un ou plusieurs scores d'état de canalisation sont associés à un ou plusieurs indices de fiabilité définissant la précision des mesures utilisées pour obtenir l'un ou plusieurs scores d'état.

8. Procédé mis en œuvre par ordinateur selon l'une des revendications 1 à 7, dans lequel l'exécution d'une estimation d'un ou plusieurs scores d'état de canalisation pour des sections de canalisation qui ne font pas partie de l'échantillon comprend :
- l'instruction, pour une catégorie, d'un moteur d'apprentissage machine supervisé qui prédit des scores d'état de canalisation sur la base des paramètres de canalisation en se servant de sections de canalisation qui font partie de l'échantillon,
- l'utilisation du dit moteur d'apprentissage machine supervisé pour prédire des scores d'état de canalisation sur la base de paramètres de canalisation des sections de canalisation de la catégorie qui ne font pas partie de l'échantillon.

9. Procédé mis en œuvre par ordinateur selon la revendication 8, dans lequel ledit moteur d'apprentissage machine supervisé est un moteur d'apprentissage machine de type forêt aléatoire.

10. Procédé mis en œuvre par ordinateur selon l'une quelconque des revendications 1 à 9, comprenant en outre l'émission d'une alerte pour des sections de canalisation dont les scores d'état de canalisation correspondent à une situation d'alerte.

11. Procédé mis en œuvre par ordinateur selon la revendication 10, dans lequel chaque alerte pour une section de canalisation déclenche automatiquement au moins une action choisie dans un groupe comprenant : une procédure d'évaluation d'état supplémentaire de la section de canalisation, une mesure de sauvegarde et une réparation de la section de canalisation.

12. Produit de programme informatique stocké dans un support non transitoire lisible par ordinateur, ledit produit de programme informatique comprenant des instructions de code qui, lorsque le programme est exécuté par un ordinateur, amènent l'ordinateur à exécuter le procédé selon l'une quelconque des revendications 1 à 11.

13. Dispositif de traitement de données comprenant un processeur configuré pour exécuter un procédé selon l'une quelconque des revendications 1 à 11.
